# EUROPEAN PATENT APPLICATION

(11) **EP 2 821 399 A1**
(43) Date of publication of application: **07.01.2015**
(21) Application number: 13175001.0
(22) Date of filing: 04.07.2013
(51) Int. Cl.: C07D 213/80, C07D 309/38, C07D 211/90, C07C 69/67

(54) **Method for preparation of 6-trifluoromethylpyridine-3-carboxylic acid derivatives from 4,4,4-trifluoro-3-oxobutanoyl chloride**

(71) Applicant: Lonza Ltd, 3930 Visp (CH)
(72) Inventor: Zaragoza Dörwald, Florencio, 3930 Visp (CH); Taeschler, Christoph, 3930 Visp (CH); Bersier, Michael, 3938 Ausserbeg (CH)

(57) **Abstract**

The invention discloses a method for the preparation of substituted 6-trifluoromethylpyridine-3-carboxylic acid derivatives starting from 4,4,4-trifluoro-3-oxobutanoyl chloride via respective intermediates and respective steps.

## Description

The invention discloses a method for the preparation of substituted 6-trifluoromethylpyridine-3-carboxylic acid derivatives starting from 4,4,4-trifluoro-3-oxobutanoyl chloride via respective intermediates and respective steps.

### BACKGROUND OF THE INVENTION

2-Trifluoromethylpyridines and 6-trifluoromethylpyridine-3-carboxylic acid derivatives are intermediates for the preparation of biologically active compounds. For instance, WO 00/39094 Al discloses trifluoromethylpyridine as herbicides, WO 2006/059103 A2 discloses trifluoromethylpyridines as intermediates in the production of pharmaceutical, chemical and agro-chemical products, WO 2008/013414 Al discloses trifluoromethylpyridines as vanilloid receptor antagonists and WO 2012/061926 Al describes trifluoromethylpyridines as calcium channel blockers.

WO 2005/026149 A1, DE 24 29 674 A1 and EP 51209 A1 disclose certain precursors used in instant invention.

The common route for the preparation of 6-trifluoromethylpyridine-3-carboxylic acid derivatives was first reported by Okada et al., Heterocycles 1997, 46, 129-132, and has only been slightly modified by others. The common synthetic strategies are summarized in Scheme 1:

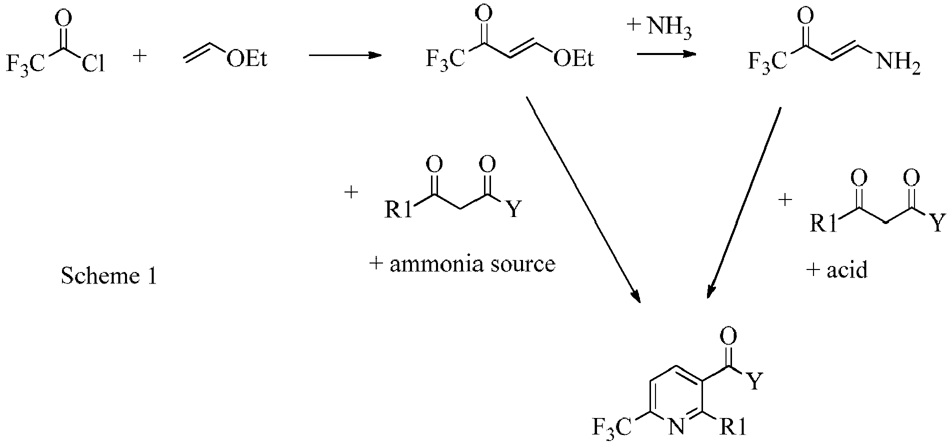

This route has disadvantages for the large scale production of 6-trifluoromethylpyridine-3-carboxylic acid derivatives, because ethylvinylether is highly flammable and therefore difficult to handle, and because the trifluoroacetylated enolether and the trifluoroacetylated enamine intermediates are unstable and cannot be stored for a longer time. Moreover, most vinyl ethers are mutagenic.

There was a need for an improved procedure for the preparation of 6-trifluoromethylpyridine-3-carboxylic acid derivatives.

This need was met by the method of instant invention as outlined below.

Compared to prior art, the method of the instant invention offers several advantages: Importantly, no vinyl ethers, trifluoroacetylated enolether intermediates or trifluoroacetylated enamine intermediates are required. Moreover, the method of the present invention only comprises few synthetic steps, what reduces the overall costs.

It was unexpected that the hydrogenolytic dehalogenation at position 4 of the pyridine substituted with halogen on position 4 was possible even with an alkoxymethyl substitutent on position 2, because usually under hydrogenation conditions benzylic C-O bonds are cleaved.

In the following text, if not otherwise stated,
- ambient pressure: usually 1 bar, depending on the weather;
- halogen: means F, Cl, Br or I, preferably Cl, Br or I;
- alkyl: means a linear or branched alkyl, examples of alkyl include methyl, ethyl, n-propyl, isopropyl, n-butyl, sec-butyl, tert-butyl, pentyl, hexyl, heptyl and the like;
- cyclic alkyl or cyclo alkyl: include cyclo aliphatic, bicyclo aliphatic and tricycle aliphatic residues; examples of "cycloalkyl" include cyclopropyl, cyclobutyl, cyclopentyl, cyclohexyl, cycloheptyl, norbornyl and adamantyl;
- alkoxy: means alkyl-O, i.e. the radical obtained by removal of the oxygen-bound hydrogen from an aliphatic alcohol;
- (alkoxy)alkoxy: refers to alkoxy groups, in which the alkyl group is substituted with one additional alkoxy group; examples of (alkoxy)alkoxy include methoxymethoxy with formula MeO-CH₂-O-, 2-(methoxy)ethoxy with formula MeO-CH₂-CH₂-O- and 2-(cyclopropylmethoxy)ethoxy with formula (C₃H₅)CH₂-O-CH₂-CH₂-O-;
- Ac: acetyl;
- tBu: tertiary butyl;
- cyanuric acid chloride: 2,4,6-trichloro-1,3,5-triazine
- DBU: 1,8-diazabicyclo[5.4.0]undec-7-ene;
- DABCO: 1,4-diazabicyclo[2.2.2]octane;
- DMF: N,N-dimethylformamide;
- DMA: N,N-dimethylacetamide;
- DMSO: dimethylsulfoxide;
- halogen: means F, Cl, Br or J, preferably F, Cl or Br;
- hemiacetal: refers to the adduct of an alcohol, for instance methanol or ethanol, with a ketone or with an aldehyde; a hemiacetal may also result upon the addition of water to an enol ether; for instance, the hemiacetal of methanol with trifluoroacetone is F₃C-C(OH)(OCH₃)-CH₃;
- hexanes: mixture of isomeric hexanes;
- hydrate: refers to the adduct of water with a ketone or with an aldehyde, for instance, the hydrate of trifluoroacetone is F₃C-C(OH)₂-CH₃;
- LDA: Lithium diisopropyl amide
- NMP: N-methyl-2-pyrrolidone;
- sulfamic acid: HO-SO₂-NH₂;
- THF: tetrahydrofuran;
- trifluoroacetone: 1,1,1-trifluoropropan-2-one;
- xylene: 1,2-dimethylbenzene, 1,3-dimethylbenzene, 1,4-dimethylbenzene or a mixture thereof.

### SUMMARY OF THE INVENTION

Subject of the invention is a method (M1) for preparation of compound of formula (I); 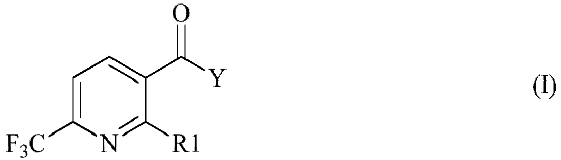 the method (M1) comprises a step (StepS1);
step (StepS1) comprises a reaction (ReacS1);
reaction (ReacS1) is a reaction of a compound of formula (II) with compound (HyS) in the presence of a catalyst (CatS1); 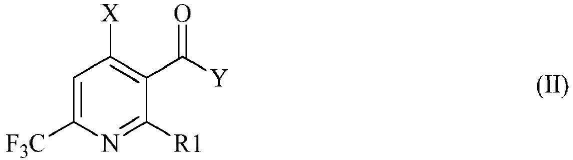 wherein
- X: is Cl or Br;
- R1: is selected from the group consisting of C₁₋₁₀ alkyl, C₃₋₈ cycloalkyl, C(O)-O-C₁₋₄ alkyl, CH=CH₂, benzyl, phenyl and naphthyl;
said C₁₋₁₀ alkyl of R1 is unsubstituted or substituted with 1, 2, 3, 4 or 5 identical or different substituents selected from the group consisting of halogen, OH, O-C(O)-C₁₋₅ alkyl, O-C₁₋₁₀ alkyl, S-C₁₋₁₀ alkyl, S(O)-C₁₋₁₀ alkyl, S(O₂)-C₁₋₁₀ alkyl, O-C₁₋₆ alkylen-O-C₁₋₆ alkyl, C₃₋₈ cycloalkyl and 1,2,4-triazolyl;
said benzyl, said phenyl and said naphthyl of R1 are independently from each other unsubstituted or substituted with 1, 2, 3, 4 or 5 identical or different substituents selected from the group consisting of halogen, C₁₋₄ alkoxy, NO₂ and CN;
- Y: is selected from the group consisting of C₁₋₆ alkoxy, O-C₁₋₆ alkylen-O-C₁₋₆ alkyl, NH₂, NHR4 and N(R4)R5;
- R4 and R5: are identical or different and independently from each other C₁₋₆ alkyl, or represent together a 1,4-butylene or a 1,5-pentylene chain;
compound (HyS) is selected from the group consisting of hydrogen, cyclohexene, tetralin, 1,2-dihydronaphthalene, 1,3-cyclohexadiene, 1,4-cyclohexadiene, hydrazine, formic acid, salts of formic acid, and mixtures thereof;
catalyst (CatS1) is a catalyst derived from palladium, ruthenium, rhodium, nickel, or platinum.

### DETAILED DESCRIPTION OF THE INVENTION

Preferably, X is Cl.

Preferably, R1 is selected from the group consisting of C₁₋₅ alkyl, C₃₋₆ cycloalkyl, C(O)-O-C₁₋₄ alkyl, CH=CH₂, benzyl and phenyl;
said C₁₋₅ alkyl of R1 is unsubstituted or substituted with 1, 2, 3, 4 or 5 identical of different substituents selected from the group consisting of halogen, O-C(O)-CH₃, O-C₁₋₅ alkyl, S-C₁₋₅ alkyl, S(O)-C₁₋₅ alkyl, S(O₂)-C₁₋₅ alkyl, O-C₁₋₄ alkylen-O-C₁₋₄ alkyl, C₃₋₆ cycloalkyl and 1,2,4-triazolyl;
said benzyl and said phenyl of R1 are independently from each other unsubstituted or substituted with 1, 2, 3, 4 or 5 identical of different substituents selected from the group consisting of halogen, C₁₋₂ alkoxy, NO₂ and CN;
more preferably, R1 is selected from the group consisting of methyl, ethyl, isopropyl, tert-butyl, cyclopropyl, trifluoromethyl, difluoromethyl, chloromethyl, bromomethyl, C(O)-O-CH₃, C(O)-O-C₂H₅, CH₂-O-C(O)-CH₃, CH₂-O-CH₃, CH₂-S-CH₃, CH₂-S(O₂)-CH₃, CH₂-CH₂-O-CH₃, CH₂-O-CH₂-CH₂-O-CH₃, CH₂-O-CH₂-CH₂-O-CH₂-CH₃, CH=CH₂ and phenyl;
even more preferably, R1 is selected from the group consisting of methyl, ethyl, isopropyl, tert-butyl, trifluoromethyl, chloromethyl, CH₂-O-CH₃, CH₂-O-CH₂-CH₂-O-CH₃ and CH₂-O-CH₂-CH₂-O-CH₂-CH₃;
especially , R1 is selected from the group consisting of methyl, chloromethyl and CH₂-O-CH₂-CH₂-O-CH₃.

Preferably, Y is selected from the group consisting of C₁₋₆ alkoxy, NHR4 and N(R4)R5;
R4 and R5 are identical or different and independently from each other C₁₋₆ alkyl, or represent together a 1,4-butylene or a 1,5-pentylene chain;
more preferably, Y is selected from the group consisting of methoxy and ethoxy.

Preferably, compound (HyS) is selected from the group consisting of hydrogen, cyclohexene, formic acid, salts of formic acid, and mixtures thereof.

Preferably, the molar amount of compound (Hys) is from 1 to 1000 fold, more preferably from 1 to 100 fold, based on the molar amount of compound of formula (II).

Preferably, salts of formic acid salts are selected from the group consisting of [N(R10)(R11)(R12)(R13)]⁺[HCOO]⁻, hydrazinium formate, sodium formate and potassium formate;

R10, R11, R12 and R13 are identical or different and independently from each other selected from the group consisting of H, C₁₋₆ alkyl, and C₂₋₆ alkylene-OH;
more preferably, salts of formic acid salts are selected from the group consisting of ammonium formate, hydrazinium formate, sodium formate, potassium formate, triethylammonium formate and tributylammonium formate.

Preferably, catalyst (CatS1) is selected from the group consisting of palladium powder, palladium on C, palladium on Al₂O₃, ruthenium on C, ruthenium on Al₂O₃, rhodium on C, rhodium on Al₂O₃, Raney nickel, platinum powder, platinum on carbon, platinum on Al₂O₃, PtO₂, and mixtures thereof.

The C in catalyst (CatS1) is preferably charcoal.

Preferably, the amount of catalyst (CatS1) is from 0.0001 to 0.1 fold, more preferably from 0.001 to 0.1 fold, even more preferably from 0.001 to 0.05 fold, based on the weight of compound of formula (II).

Preferably, reaction (ReacS1) is done in a solvent (SolvS1) solvent (SolvS1) is selected from the group consisting of methanol, ethanol, 2-methoxyethanol, propanol, isopropanol, butanol, 2-butanol, isobutanol, water, ethyl acetate, butyl acetate, THF, 2-methyl-THF, 3-methyl-THF, benzene, toluene, xylene, acetic acid, propionic acid, and mixtures thereof.

Preferably, the weight of solvent (SolvS1) is from 0.1 to 100 times, more preferably from 1 to 50 times, even more preferably from 1 to 10 times, of the weight of compound of formula (II).

Preferably, reaction (ReacS1) is done in the presence of a base (BasS1); base (BasS1) is selected from the group consisting of N(R20)(R21)(R22), unsubstituted pyridine, pyridine substituted with 1, 2 and 3 identical or different substituents independently from each other selected from the group consisting of methyl and ethyl, salts of [N(R30)(R31)(R32)(R33)]⁺ with C₂₋₆ carboxylic acids, salts of Na and K with carbonate and bicarbonate, hydroxides of Na, K and Ca, and mixtures thereof;
R20, R21 and R22 are identical or different and independently from each other selected from the group consisting of H, C₁₋₆ alkyl and C₂₋₆ alkylene-OH;
R30, R31, R32 and R33 are identical or different and independently from each other H or C₁₋₆ alkyl;
more preferably, base (BasS1) is selected from the group consisting of triethylamine, tributylamine, triethanolamine, pyridine, 2-methylpyridine, 3-methylpyridine, 4-methylpyridine, 2-methyl-5-ethylpyridine, 2,4,6-trimethylpyridine, ammonia, ammonium acetate, sodium carbonate, sodium bicarbonate, potassium carbonate, potassium bicarbonate, calcium hydroxide, and mixtures thereof;
even more preferably, base (BasS1) is selected from the group consisting of triethylamine, tributylamine, triethanolamine, ammonia, ammonium acetate, sodium carbonate, sodium bicarbonate, potassium carbonate, potassium bicarbonate, calcium hydroxide, and mixtures thereof.

Preferably, the molar ratio [base (BasS1) : compound of formula (II)] is from [0.1 : 1] to [10 : 1], more preferably from [0.3 : 1] to [5 : 1], even more preferably from [0.5 : 1] to [4 : 1].

Preferably, reaction (ReacS1) is done at a temperature of from 0 to 200°C, more preferably from 10 to 150°C, even more preferably from 20 to 130°C.

Preferably, reaction (ReacS1) is done at a pressure of from 0.5 to 20 bar, more preferably from ambient pressure to 10 bar, even more preferably from ambient pressure to 5 bar.

Preferably, reaction time of reaction (ReacS1) is from 10 min to 72 h, more preferably from 1 h to 48 h, even more preferably from 2 h to 24 h.

After reaction (ReacS1), the catalyst (CatS1) is preferably removed by filtration, and compound of formula (I) may be isolated by any conventional method. If the product spontaneously crystallizes, it can be isolated by filtration of the filtered reaction mixture. Preferably, any solvent is distilled off, and the residue is purified or used without further purification.

Alternatively, the residue is recrystallized from a suitable solvent, such as benzene, toluene, C₅-C₁₀ alkanes, or mixtures thereof.

Alternatively, the residue is mixed with water, the pH is adjusted to 0 to 12, preferably to 2 to 10, more preferably to 3 to 8, by addition of an acid or a base, the acid is preferably HCl or H₂SO₄, the base is preferably selected from the group consisting of NaOH, KOH, Ca(OH)₂, Na₂CO₃, NaHCO₃, and K₂CO₃, and compound of formula (I) is either isolated by filtration or extracted with a solvent such as ethyl acetate, isopropyl acetate, butyl acetate, C₅₋₁₀ alkanes, toluene, chlorobenzene, dichloromethane, chloroform, or mixtures thereof; after distilling off the solvent used for the extraction, the crude product may be further purified by recrystallization or distillation.

Preferably, method (M1) comprises furthermore a step (S2), step (S2) is done before step (S1), in step (S2) compound of formula (II) is prepared;
step (StepS2) comprises a reaction (ReacS2);
reaction (ReacS2) is a reaction of a compound of formula (III) with a compound (HalS2); compound of formula (III) is selected from the group consisting of compound of formula (IIIa), compound of formula (IIIb) and mixtures thereof; 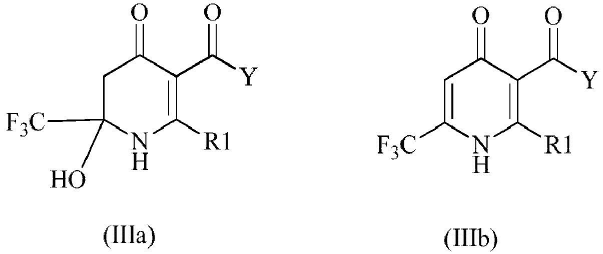 with R1 and Y as defined above, also with all their preferred embodiments;
compound (HalS2) is selected from the group consisting of POCl₃, PCl₃, PCl₅, SOCl₂, COCl₂, diphosgene, triphosgene, (COCl)₂, 2,4,6-trichloro-1,3,5-triazine, POBr₃, PBr₃, PBr₅, Ph₃PBr₂, Br₂ with P(OPh)₃, P₄O₁₀ together with NaBr, N-bromosuccinimide together with PPh₃, and mixtures thereof.

Compound (HalS2) is chosen according to the desired X, i.e. either X is Cl or X is Br.

Therefore when X shall be is Cl, then compound (HalS2) is selected from the group consisting of POCl₃, PCl₃, PCl₅, SOCl₂, COCl₂, diphosgene, triphosgene, (COCl)₂, 2,4,6-trichloro-1,3,5-triazine, and mixtures thereof;
whereas when X shall be is Br, then compound (HalS2) is selected from the group consisting of POBr₃, PBr₃, PBr₅, Ph₃PBr₂, Br₂ with P(OPh)₃, P₄O₁₀ together with NaBr, N-bromosuccinimide together with PPh₃, and mixtures thereof.
But chlorinating and brominating compounds (HalS2) can also be used together, resulting in a mixture of compounds of formula (II) with X being Cl and Br respectively.

Preferably, in case that X shall be Cl, then compound (HalS2) is selected from the group consisting of POCl₃, PCl₃, PCl₅, SOCl₂, COCl₂, diphosgene, triphosgene, (COCl)₂, and mixtures thereof.

Preferably, in case that X shall be Br, then compound (HalS2) is selected from the group consisting of, POBr₃, PBr₃, PBr₅, Ph₃PBr₂, Br₂ with P(OPh)₃, N-bromosuccinimide together with PPh₃, and mixtures thereof;
more preferably, compound (HalS2) is selected from the group consisting of POBr₃, PBr₃, PBr₅, Ph₃PBr₂, Br₂ with P(OPh)₃, and mixtures thereof.

Preferably, the molar amount of compound (HalS2) is from 1 to 100 fold, more preferably from 1 to 50 fold, even more preferably from 1 to 10 fold, based on the molar amount of compound of formula (III).

Preferably, reaction (ReacS2) is done in a solvent (SolvS2); solvent (SolvS2) is selected from the group consisting of acetonitrile, propionitrile, benzene, toluene, xylene, chlorobenzene, anisole, pyridine, 2-methylpyridine, 3-methylpyridine, 4-methylpyridine, 2-methyl-5-ethylpyridine, ethyl acetate, THF, 2-methyl-THF, 3-methyl-THF, chloroform, dichloromethane, 1,2-dichloroethane, 1,2-dichloropropane, and mixtures thereof;
more preferably, solvent (SolvS2) is selected from the group consisting of benzene, toluene, xylene, chlorobenzene, pyridine, 3-methylpyridine, chloroform, dichloromethane, 1,2-dichloroethane, 1,2-dichloropropane, and mixtures thereof.

Preferably, the weight of solvent (SolvS2) is from 0.1 to 100 times, more preferably from 1 to 50 times, even more preferably from 1 to 10 times, of the weight of compound of formula (III).

Reaction (ReacS2) can be done in the presence of a base (BasS2); base (BasS2) is selected from the group consisting of N,N-di-C₁₋₄ alkyl aniline, N(R40)(R41)(R42), N-C₁₋₄ alkyl morpholine, unsubstituted pyridine, pyridine substituted with 1, 2 and 3 identical or different substituents independently from each other selected from the group consisting of methyl and ethyl, and mixtures thereof;

R40, R41 and R42 are identical or different and independently from each other C₁₋₆ alkyl; more preferably, base (BasS2) is selected from the group consisting of N,N-dimethylaniline, triethylamine, tributylamine, N-methylmorpholine, N-ethylmorpholine, pyridine, 2-methylpyridine, 3-methylpyridine, 4-methylpyridine, 2-methyl-5-ethylpyridine, 2,4,6-trimethylpyridine, and mixtures thereof.

Preferably, the molar ratio of [base (BasS2) : compound of formula (III)] is from [10 : 1] to [1 : 10], more preferably from [5 : 1] to [1 : 5], and most preferably from [3 : 1] to [1 : 3].

Preferably, reaction (ReacS2) is done at a temperature of from 0 to 200°C, more preferably from 10 to 150°C, even more preferably from 20 to 130°C.

Preferably, reaction (ReacS2) is done at a pressure of from 0.5 to 20 bar, more preferably from ambient pressure to 10 bar, even more preferably from ambient pressure to 5 bar.

Preferably, reaction time of reaction (ReacS2) is from 1 min to 72 h, more preferably from 5 min to 48 h, even more preferably from 5 min to 24 h.

After reaction (ReacS2), compound of formula (II) may be isolated by any conventional method. If compound of formula (II) spontaneously crystallizes, it can be isolated by filtration. Preferably, any solvent or residual compound (HalS2) is distilled off, and the crude residue is used for the next reaction without further purification.

Alternatively, the crude residue is recrystallized from a suitable solvent, such as benzene, toluene or C₅-₁₀ alkanes.

Alternatively, the crude residue is mixed with water, the pH is adjusted to 0 to 12, preferably to 2 to 10, more preferably to 3 to 8, by addition of an acid or a base, the acid is preferably HCl or H₂SO₄, the base is preferably selected from the group consisting of NaOH, KOH, Ca(OH)₂, Na₂CO₃, NaHCO₃, and K₂CO₃, and compound of formula (II) is either isolated by filtration or extracted with a solvent such as ethyl acetate, isopropyl acetate, butyl acetate, toluene, chlorobenzene, dichloromethane or chloroform, and after distilling off the solvent used for the extraction the crude product may be further purified by recrystallization.

Preferably, method (M1) comprises furthermore a step (S3), step (S3) is done before step (S2), in step (S3) compound of formula (III) is prepared;
step (StepS3) comprises a reaction (ReacS3);
reaction (ReacS3) is a reaction of a compound of formula (IV) with a compound (AMS);
compound of formula (IV) is selected from the group consisting of compound of formula (IVa), compound of formula (IVb), compound of formula (IVc), a hydrate of compound of formula (IVa), of compound of formula (IVb) and of compound of formula (IVc), and mixtures thereof; 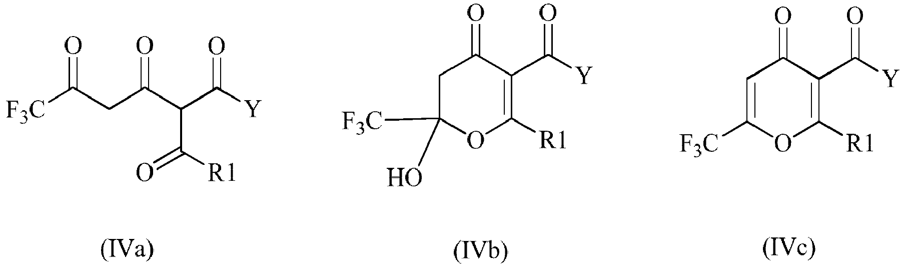 with R1 and Y as defined above, also with all their preferred embodiments;
compound (AMS) is selected from the group consisting of NH₃, sulfamic acid, urea, formamide, NH₂-C(O)-C₁₋₄ alkyl, ammonium carbonate, ammonium bicarbonate, ammonium salts of C₁₋₆ carboxylic acids, ammonium chloride, ammonium bromide, and mixtures thereof.

Preferably, compound (AMS) is selected from the group consisting of NH₃, formamide, ammonium carbonate, ammonium bicarbonate, ammonium formate, ammonium acetate, ammonium chloride and mixtures thereof.

Preferably, the molar amount of compound (AMS) is from 1 to 100 fold, more preferably from 1 to 50 fold, even more preferably from 1 to 10 fold, based on the molar amount of compound of formula (IV).

Preferably, reaction (ReacS3) is done in a solvent (SolvS3);
solvent (SolvS3) is selected from the group of C₁₋₆ alkanol, acetonitrile, propionitrile, C₁₋₄ carboxylic acid, benzene, toluene, xylene, chlorobenzene, anisole, pyridine, 2-methylpyridine, 3-methylpyridine, 4-methylpyridine, 2-methyl-5-ethylpyridine, DMF, DMA, DMSO, water, ethyl acetate, acetone, methylethylketone, THF, 2-methyl-THF, 3-methyl-THF, chloroform, dichloromethane, 1,2-dichloroethane, 1,2-dichloropropane, 1,2-dimethoxyethane, diethyleneglycoldimethylether, and mixtures thereof;
more preferably, solvent (SolvS3) is selected from the group consisting of C₁-₆ alkanol, acetonitrile, propionitrile, C₁-₃ carboxylic acid, toluene, xylene, chlorobenzene, 3-methylpyridine, 2-methyl-5-ethylpyridine, DMF, DMA, methylethylketone, chloroform, dichloromethane, 1,2-dichloroethane, 1,2-dichloropropane, water, and mixtures thereof.

Preferably, the weight of solvent (SolvS3) is from 0.1 to 100 times, more preferably from 0.25 to 50 times, even more preferably from 0.5 to 10 times, of the weight of compound of formula (IV).

Preferably, reaction (ReacS3) is done at a temperature of from 0 to 200°C, more preferably from 10 to 150°C, even more preferably from 20 to 130°C.

Preferably, reaction (ReacS3) is done at a pressure of from 0.5 to 20 bar, more preferably from ambient pressure to 10 bar, even more preferably from ambient pressure to 5 bar.

Preferably, reaction time of reaction (ReacS3) is from 10 min to 72 h, more preferably from 20 min h to 48 h, even more preferably from 30 min to 24 h.

After reaction (ReacS3), compound of formula (III) may be isolated by any conventional method. If the product spontaneously crystallizes, it can be isolated by filtration. Preferably, any solvent is distilled off, the residue is diluted with water, the pH is adjusted to 0 to 12, preferably to 2 to 10, more preferably to 3 to 8, by addition of an acid or a base, the acid is preferably HCl or H₂SO₄, the base is preferably selected from the group consisting of NaOH, KOH, Ca(OH)₂, Na₂CO₃, NaHCO₃, and K₂CO₃, and compound of formula (III) is either isolated by filtration or extracted with a solvent such as ethyl acetate, isopropyl acetate, butyl acetate, toluene, chlorobenzene, dichloromethane or chloroform, and after distilling off the solvent used for the extraction the crude product may be further purified by recrystallization.

Compound of formula (IIIb) is the dehydrated form of compound of formula (IIIa). When a solvent (SolvS3) is used and when it is a C₁₋₆ alkanol, or when compound (AMS) is an ammonium salt of a C₁₋₆ carboxylic acid, then compound of formula (III) can also be a compound of formula (IIIc), which is an adduct of the C₁₋₆ alkanol to compound of formula (IIIb) or a reaction product of compound of formula (IIIa) or of compound of formula (IIIb) with a C₁₋₆ carboxylic acid; 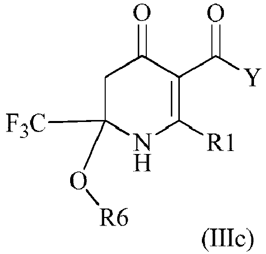 R6 is C₁₋₆ alkyl or C(O)-C₁₋₆ alkyl.

Anyone of the compounds of formula (IIIa), of formula (IIIb) and of formula (IIIc) reacts in reaction (ReacS2).

Preferably, method (M1) comprises furthermore a step (S4), step (S4) is done before step (S3), in step (S4) compound of formula (IV) is prepared;
step (StepS4) comprises a reaction (ReacS4);
reaction (ReacS4) is a reaction of a compound of formula (V) with a compound of formula (VI) facilitated by the use of a compound (SalS4); 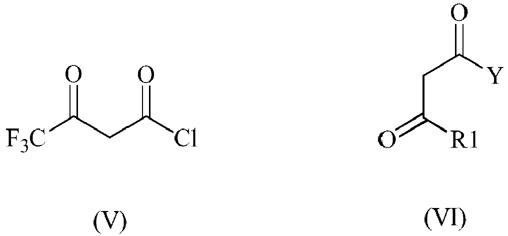 with R1 and Y as defined above, also with all their preferred embodiments;
compound (SalS4) is selected from the group consisting of NaH, Na(O(C₁₋₆ alkyl)), CaH₂, CaCl₂, MgCl₂, MgBr₂, MgI₂, Mg(O(C₁₋₆ alkyl))₂, TiCl₄, TiCl₂(O(C₁₋₆ alkyl))₂, TiCl₃(O(C₁₋₆ alkyl)), ZnCl₂, ZnBr₂, ZnI₂, SnCl₄, SnBr₄, AlCl₃, AlBr₃, Al(O(C₁₋₆ alkyl))₃, and mixture thereof.

Preferably, the molar ratio [compound of formula (V) : compound of formula (VI)] is from [1 : 5.1] to [5.1 : 1], more preferably from [1 : 3.1] to [3.1 : 1], even more preferably from [1 : 2.1] to [2.1 : 1].

Preferably, compound (SalS4) is selected from the group consisting of Na(O(C₁₋₆ alkyl)), CaH₂, CaCl₂, MgCl₂, MgBr₂, Mg(O(C₁₋₆ alkyl))2, TiCl₄, TiCl₂(O(C₁₋₆ alkyl))2, TiCl₃(O(C₁₋₆ alkyl)), ZnCl₂, AlCl₃, AlBr₃, Al(O(C₁₋₆ alkyl))₃, and mixture thereof;
more preferably, compound (SalS4) is selected from the group consisting of MgCl₂, MgBr₂, Mg(O(C₁₋₆ alkyl))2, AlCl₃, AlBr₃, Al(O(C₁₋₆ alkyl))₃, and mixture thereof.
Preferably, the molar ratio [salt (SalS4) compound of formula (VI)] is from [0.1 : 1] to [10 : 1], more preferably from [0.3 : 1] to [5 : 1], even more preferably from [0.75 : 1] to [4 : 1].

Preferably, reaction (ReacS4) is done in a solvent (SolvS4);
solvent (SolvS4) is selected from the group consisting of THF, 2-methyl-THF, 3-methyl-THF, chloroform, dichloromethane, 1,2-dichloroethane, 1,2-dichloropropane, chlorobenzene, DMA, acetonitrile, propionitrile, 1,2-dimethoxyethane, methoxycyclopentane, diethyleneglycoldimethylether, diethyleneglycoldiethylether, benzene, toluene, anisole, and mixtures thereof;
preferably, solvent (SolvS4) is selected from the group consisting of THF, 2-methyl-THF, 3-methyl-THF, chloroform, dichloromethane, 1,2-dichloroethane, 1,2-dichloropropane, chlorobenzene, 1,2-dimethoxyethane, benzene, toluene, and mixtures thereof;
more preferably, solvent (SolvS4) is selected from the group consisting of dichloromethane, 1,2-dichloroethane, chlorobenzene, toluene, and mixtures thereof.
Preferably, the weight of solvent (SolvS4) is from 0.1 to 100 times, more preferably from 1 to 50 times, of the weight of compound of formula (V).

Preferably, reaction (ReacS4) is facilitated by the use of a base (BasS4); base (BasS4) is selected from the group consisting of N(R50)(R51)(R52), unsubstituted pyridine, pyridine substituted with 1, 2 and 3 identical or different substituents independently from each other selected from the group consisting of methyl and ethyl, N-C₁₋₄ alkyl morpholine, and mixtures thereof;
R50, R51 and R52 are identical or different and independently from each other C₁₋₆ alkyl;
more preferably, base (BasS4) is selected from the group consisting of triethylamine, tributylamine, ethyldiisopropylamine, N-methylmorpholine, pyridine, 3-methylpyridine, 2-methyl-5-ethylpyridine, 2,4,6-trimethylpyridine, and mixtures thereof;
even more preferably, base (BasS4) is selected from the group consisting of triethylamine, tributylamine, pyridine, 3-methylpyridine, and mixtures thereof.

Preferably, the molar ratio [base (BasS4) : compound of formula (V)] is from [0.1 : 1] to [10 : 1], more preferably from [0.3 : 1] to [5 : 1], even more preferably from [0.5 : 1] to [4 : 1].

Preferably, reaction (ReacS4) is done at a temperature of from -70 to 150°C, more preferably from -50 to 120°C, even more preferably from -40 to 100°C.

Preferably, reaction (ReacS4) is done at a pressure of from 1 to 20 bar, more preferably of from ambient pressure to 15 bar, even more preferably of from ambient pressure to 10 bar.

Preferably, the reaction time of reaction (ReacS4) is from 30 min to 96 h, more preferably from 1 h to 48 h, even more preferably from 2 h to 36 h.

Preferably, in reaction (ReacS4) compound of formula (VI) is reacted in a first reaction (ReacS4a) with compound (SalS4) resulting in an intermediate (IntS4), followed by a reaction (ReacS4b) of intermediate (IntS4) with compound (V).

Intermediate (IntS4) can be used without isolation after reaction (ReacS4a) for the reaction (ReacS4b), or it is isolated by any conventional method before reaction (ReacS4b), preferably by evaporation of any solvent or by crystallization.

In one more preferable embodiment, reaction (ReacS4) is done by the addition of compound of formula (V) to a mixture of compound of formula (VI), compound (SalS4) with solvent (SolvS4).

In another more preferable embodiment, reaction (ReacS4) is done by the addition of compound of formula (V) to a mixture of intermediate (IntS4) with solvent (SolvS4).

After reaction (ReacS4), compound of formula (IV) may be isolated by any conventional method. Preferably, compound of formula (IV) is isolated after reaction (ReacS4) by hydrolysis and acidification of the reaction mixture resulting from reaction (ReacS4). Hydrolysis and acidification is preferably done by addition of a compound (InAcS4), compound (InAcS4) is an aqueous inorganic acid, preferably compound (InAcS4) is selected from the group consisting of aqueous hydrochloric acid and aqueous sulfuric acid.

After hydrolysis and acidification, any solvent (SolvS4) is preferably removed by distillation, followed by extraction with a solvent (SolvExtrS4), solvent (SolvExtrS4) is preferably selected from the group consisting of ethyl acetate, isopropyl acetate, butyl acetate, toluene, chlorobenzene, dichloromethane, chloroform and mixtures thereof; the extraction is preferably followed by removal of solvent (SolvExtrS4) by distillation.

The use of water during isolation of compound of formula (IV) can lead to the formation of a hydrate of compound of formula (IV), such as to the formation of a hydrate of compound of formula (IVa), of compound of formula (IVb) or of compound of formula (IVc).

Compound of formula (IVc) is the dehydrated form of compound of formula (IVb).

The ratio of compound of formula (IVa) : compound of formula (IVb) depends on the nature of R1 and Y, on the nature of solvent (SolS4) and on conditions of isolation, such as the nature of a solvent (SolveExtrS4) and temperature. The occurrence of compound of formula (IVc) is favored under elevated temperature and dehydrating conditions. Anyone of compound of formula (IVa), of formula (IVb) and of formula (IVc) reacts in reaction (ReacS3).

The compound of formula (III), compound of formula (IV), compound of formula (V) and compound of formula (VI) comprises also any of the possible tautomeric forms.

Compound of formula (V) and compound of formula (VI) are known compound and can be prepared according to known methods.

Further subject of the invention is a method (M2) for preparation of compound of formula (II);
the method (M2) comprises the step (S2),
wherein compound of formula (II) and step (S2) are defined as above, also with all their preferred embodiments.
Preferably, method (M2) comprises furthermore the step (S3), step (S3) is done before step (S2), in step (S3) compound of formula (III) is prepared;
wherein compound of formula (III) and step (S3) are defined as above, also with all their preferred embodiments.
Preferably, method (M2) comprises furthermore the step (S4), step (S4) is done before step (S3), in step (S4) compound of formula (IV) is prepared;
wherein compound of formula (IV) and step (S4) are defined as above, also with all their preferred embodiments.
Further subject of the invention is a method (M3) for preparation of compound of formula (III);
the method (M3) comprises the step (S3),
wherein compound of formula (III) and step (S3) are defined as above, also with all their preferred embodiments.
Preferably, method (M3) comprises furthermore the step (S4), step (S4) is done before step (S3), in step (S4) compound of formula (IV) is prepared;
wherein compound of formula (IV) and step (S4) are defined as above, also with all their preferred embodiments.

Further subject of the invention is a method (M4) for preparation of compound of formula (IV);
the method (M4) comprises the step (S4),
wherein compound of formula (IV) and step (S4) are defined as above, also with all their preferred embodiments.
Further subject of the invention are compound of formula (IV), compound of formula (III) and compound of formula (II);
wherein compound of formula (IV), compound of formula (III) and compound of formula (II) are defined as above, also with all their preferred embodiments.

Further subject of the invention are compound of formula (IV), compound of formula (III) and compound of formula (II);
wherein
compound of formula (IV) is compound of formula (IVc-1);
compound of formula (III) is compound of formula (IIIa-1) or compound of formula (IIIb-1); and
compound of formula (II) is compound of formula (II-1).

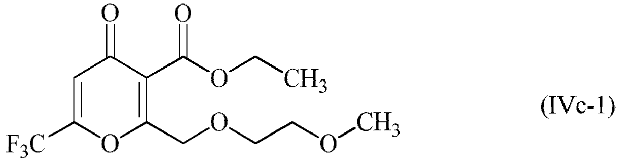

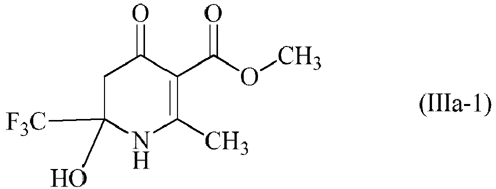

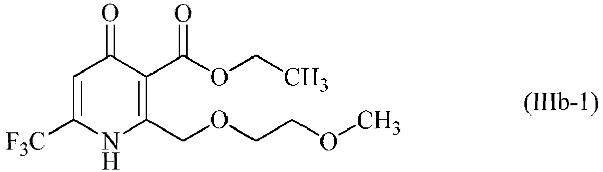

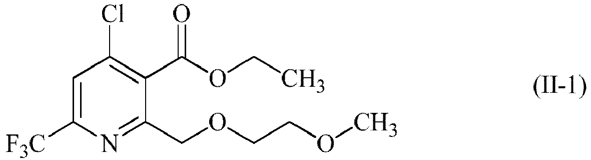

Further subject of the invention is the use of anyone of compound of formula (IV), compound of formula (III), compound of formula (II) and compound of formula (I), for the preparation of pharmaceutical, chemical or agro-chemical products,
with compound of formula (IV), compound of formula (III), compound of formula (II) and compound of formula (I) as defined above, also with all their preferred embodiments,
wherein compound of formula (IV), compound of formula (III), compound of formula (II) and compound of formula (I) have been prepared according to one of the respective methods as defined above, also with all their preferred respective embodiments.

### EXAMPLES

### Example 1: Reaction (ReacS4) and reaction (ReacS3)

A mixture of magnesium methyl acetoacetate (155 mg, 1.2 mmol; prepared from magnesium methoxide and methyl acetoacetate similarly as described in example V of EP 51209 A1), acetonitrile (1.54 ml), and triethylamine (0.314 ml, 2.4 mmol) was stirred at room temperature for 1 h 20 min. The mixture was then cooled to 0°C, and a 20% (w/w) solution of 4,4,4-trifluoro-3-oxobutanoyl chloride in 1,2-dichloroethane (0.8 ml, ca. 1.2 mmol) was added. Stirring at 0°C was continued for 1 h, and then the mixture was stirred at room temperature for 21 h. The mixture was hydrolized by addition of 1N aqueous HCl (5 ml), and then extracted with ethyl acetate (3 ml). The extract was concentrated, and the residue was mixed with MeOH (2 ml) and 25% (w/w) aqueous ammonia (1.5 ml). The solution was kept at room temperature for 24 h, and was then evaporated to dryness. The remaining solid was recrystallized from acetonitrile, to yield 126 mg (0.5 mmol, 42%) of compound of formula (IIIa-1).

¹H NMR (400 MHz, d₆-DMSO; main isomer) delta 2.16 (s, 3H), 3.72 (s, 3H), 4.43 (d, J = 1 Hz, 1H), 5.99 (d, J = 1 Hz, 1H), 6.44 (s, 1H).

GC-MS: one main compound; m/z (%) 235 (55; M - H₂O), 203 (100; 235 - MeOH), 175 (64).

### Example 2: Reaction (ReacS4)

4.66 g of anhydrous magnesium chloride (0.049 mol, 1 eq.) were mixed with CH₂Cl₂ (69 g, 0.83 mol). To this mixture 10 g of ethyl 4-(2-methoxyethoxy)acetoacetate (0.049 mol, 1 eq., which can be prepared according to Intermediate 123 in WO 2005/026149 A1) were added drop wise. The mixture was then cooled to 0°C and 7.75 g of pyridine (0.097 mol, 2 eq.) were added drop wise in order to maintain the temperature below 2°C. The mixture was stirred at 0°C for 30 min and cooled to -10°C. Then 26.7 g of a solution of 4,4,4-trifluoroacetoacetyl chloride in dichloroethane (64 wt%, 0.097 mol, 2 eq., prepared according to example 1 of DE 24 29 674 A1) were added drop wise in order to maintain the temperature below -9°C. The reaction mixture was stirred for 14 h at -10°C. The reaction mixture was heated to -5°C and quenched by addition of 100 g of 1 M aqueous HCl solution. The water phase was discarded. The organic phase was then washed with 50 g of water, dried over sodium sulphate and concentrated under reduced pressure providing the crude product as a brown oil.

Flash chromatography with Biotage® Isolera™ (Gradient Heptane/AcOEt: 1 column volume with 7% (v/v) AcOEt, change from 7% to 49% (v/v) AcOEt during 7 column volumes) afforded 9.38 g of compound of formula (IVc-1) (56%) as a brownish oil.

¹H NMR (400 MHz, DMSO-d₆) delta ppm 1.26 (t, J=7.09 Hz, 3 H) 3.22 (s, 3 H) 3.42 to 3.45 (m, 2 H) 3.59 to 3.64 (m, 2 H) 4.26 (q, J=7.11 Hz, 2 H) 4.54 (s, 2 H) 7.18 (s, 1 H)

¹³C NMR (100 MHz, DMSO-d₆) delta ppm 13.72 (s, 1 C), 57.98 (s, 1 C), 61.62 (s, 1 C), 67.14 (s, 1 C), 70.21 (s, 1 C), 70.99 (s, 1 C), 115.22 (s, 1 C), 116.96 (q, J=274 Hz, 1 C), 122.80 (s, 1 C), 151.09 (q, J=40 Hz, 1 C), 162.22 (s, 1 C), 164.16 (s, 1 C), 173.77 (s, 1 C)

¹⁹F (376 MHz, DMSO-d₆) delta ppm -70.30 (s, 3 F)

### Example 3: Reaction (ReacS3)

2.0 g of compound of formula (IVc-1), prepared according example 2 (0.006 mol, 1 eq.), were dissolved in 0.27 g of EtOH. 1.15 g of a solution of ammonia in EtOH (13 wt%, 0.008 mol, 1.5 eq.) were added at 22°C. An exothermic reaction took place and the internal temperature reached 40°C. After 1 h stirring at 30 to 40°C, the reaction mixture was cooled to -5°C. The product crystallized. Ethanol (0.4 g), that had a temperature of ca. -5°C, was added to the reaction mixture. The solid was filtered off, washed with EtOH (0.5 g), that had a temperature of -5°C, and dried for 4 h under reduced pressure (20 mbar). Compound of formula (IIIb-1) (1.79 g, 90%) was isolated as a yellow solid.

¹H NMR (400 MHz, CDCl₃) delta ppm 1.47 (t, J=7.15 Hz, 3 H) 3.37 (s, 3 H) 3.53 to 3.59 (m, 2 H) 3.67 to 3.74 (m, 2 H) 4.52 (q, J=7.07 Hz, 2 H) 4.95 (s, 2 H) 7.23 (s, 1 H)

¹³C NMR (100 MHz, CDCl₃) delta ppm 13.95 (s, 1 C), 58.96 (s, 1 C), 62.99 (s, 1 C), 70.40 (s, 1 C), 71.79 (s, 1 C), 73.95 (s, 1 C), 110.11 (s, 1 C), 112.03 (s, 1 C), 112.11 (q, J=273 Hz, 1 C), 149.69 (q, J=35 Hz, 1 C), 161.19 (s, 1 C), 169.24 (s, 1 C), 169.39 (s, 1 C)

¹⁹F (376 MHz, CDCl₃) delta ppm -69.17 (s, 3 F)

### Example 4: Reaction (ReacS2)

Compound of formula (IIIb-1) (0.16 g, 0.49 mmol, 1 eq.)), prepared according example 3, were dissolved in POCl₃ (0.71 g, 1.96 mol, 4 eq.). The reaction mixture was stirred at 100°C for 10 min in a microwave apparatus (Biotage^{®} Initiator 8). The reaction was quenched by addition of 5 g of water and diluted with 3 g of CH₂Cl₂. The water phase was discarded. The organic phase was dried over sodium sulphate and concentrated under reduced pressure providing the crude product as a residual oil.

Flash chromatography with Biotage® Isolera™ (Gradient Heptane/AcOEt: 1 column volume with 5% (v/v) AcOEt, 5% to 56% (v/v) AcOEt during 9 column volumes) afforded 0.14 g of compound of formula (II-1) (90%) as a light-yellow oil.

¹H NMR (400 MHz, CDCl₃) delta ppm 1.42 (t, J=7.15 Hz, 3 H) 3.37 (s, 3 H) 3.50 to 3.57 (m, 2 H) 3.60 to 3.65 (m, 2 H) 4.46 (q, J=7.15 Hz, 2 H) 4.83 (s, 2 H) 7.68 (s, 1 H)

¹³C NMR (100 MHz, DMSO-d₆) delta ppm 13.94 (s, 1 C) 58.96 (s, 1 C) 62.46 (s, 1 C) 70.52 (s, 1 C) 71.55 (s, 1 C) 73.02 (s, 1 C) 120.86 (q, J=2.93 Hz, 1 C) 121.81 (q, J=272 Hz, 1 C) 131.35 (s, 1 C) 143.66 (s, 1 C) 148.20 (q, J=34.48 Hz, 9 C) 158.59 (s, 1 C) 164.33 (s, 1 C)

¹⁹F (376 MHz, CDCl₃) delta ppm -68.33 (s, 3 F)

### Example 5: Reaction (ReacS1)

Compound of formula (II-1) (0.03 g, 0.087 mmol, 1 eq.), prepared according example 4, was dissolved in 0.17 g of EtOH. Triethylamine (0.011 g, 0.13 mmol, 1.5 Eq.) was then added. Then 0.003 g of 10% (w/w) Pd/C (as 50% (w/w) water wet paste) was added and the reaction mixture was hydrogenated with H₂ using a balloon for 2 h at ambient temperature. The reaction mixture was filtered over celite and the filter cake was washed with 5 g of EtOH. The solvent was evaporated under reduced pressure providing the crude product as a light-yellow oil.

Flash chromatography with Biotage® Isolera™ (Gradient Heptane/AcOEt: 1 column volume with 5% (v/v) AcOEt, 5% to 39% (v/v) AcOEt during 9.7 column volumes) afforded 0.026 g of compound of formula (I-1) (95%) as a light-yellow oil.

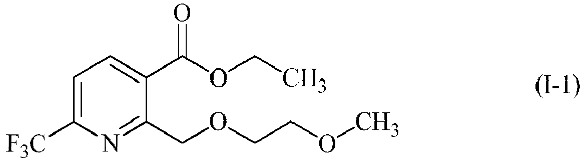

¹H NMR (400 MHz, CDCl₃) delta ppm 1.42 (t, J=7.15 Hz, 3 H) 3.36 (s, 3 H) 3.54 to 3.58 (m, 2 H) 3.68 to 3.74 (m, 2 H) 4.43 (q, J=7.15 Hz, 2 H) 5.02 (s, 2 H) 7.68 (d, J=8.03 Hz, 1 H) 8.26 (d, J=8.03 Hz, 1 H)

¹³C NMR (100 MHz, CDCl₃) delta ppm 14.11 (s, 1 C) 58.96 (s, 1 C) 62.05 (s, 1 C) 70.56 (s, 1 C) 71.75 (s, 1 C) 72.89 (s, 1 C) 119.19 (q, J=2.7 Hz, 1 C) 119.65 (q, J=273 Hz, 1 C) 129.17 (s, 1 C) 139.40 (s, 1 C) 148.80 (q, J=35 Hz, 9 C) 158.03 (s, 1 C) 165.62 (s, 1 C)

¹⁹F (376 MHz, CDCl₃) delta ppm -68.24 (s, 3 F)

## Claims

1. Method (M1) for preparation of compound of formula (I); 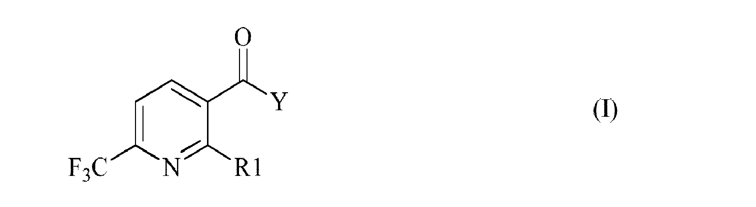 the method (M1) comprises a step (StepS1);
step (StepS1) comprises a reaction (ReacS1);
reaction (ReacS1) is a reaction of a compound of formula (II) with compound (HyS) in the presence of a catalyst (CatS1); 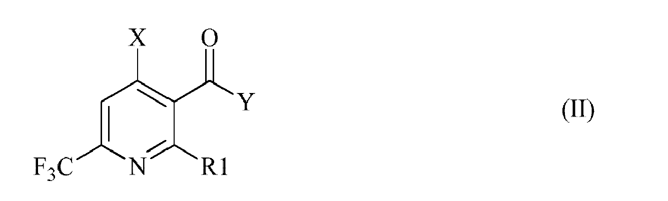 wherein
X is Cl or Br;
R1 is selected from the group consisting of C₁₋₁₀ alkyl, C₃₋₈ cycloalkyl, C(O)-O-C₁₋₄ alkyl, CH=CH₂, benzyl, phenyl and naphthyl;
said C₁₋₁₀ alkyl of R1 is unsubstituted or substituted with 1, 2, 3, 4 or 5 identical or different substituents selected from the group consisting of halogen, OH, O-C(O)-C₁₋₅ alkyl, O-C₁₋₁₀ alkyl, S-C₁₋₁₀ alkyl, S(O)-C₁₋₁₀ alkyl, S(O₂)-C₁₋₁₀ alkyl, O-C₁₋₆ alkylen-O-C₁₋₆ alkyl, C₃₋₈ cycloalkyl and 1,2,4-triazolyl;
said benzyl, said phenyl and said naphthyl of R1 are independently from each other unsubstituted or substituted with 1, 2, 3, 4 or 5 identical or different substituents selected from the group consisting of halogen, C₁₋₄ alkoxy, NO₂ and CN;
Y is selected from the group consisting of C₁₋₆ alkoxy, O-C₁₋₆ alkylen-O-C₁₋₆ alkyl, NH₂, NHR4 and N(R4)R5;
R4 and R5 are identical or different and independently from each other C₁₋₆ alkyl, or represent together a 1,4-butylene or a 1,5-pentylene chain;
compound (HyS) is selected from the group consisting of hydrogen, cyclohexene, tetralin, 1,2-dihydronaphthalene, 1,3-cyclohexadiene, 1,4-cyclohexadiene, hydrazine, formic acid, salts of formic acid, and mixtures thereof;
catalyst (CatS1) is a catalyst derived from palladium, ruthenium, rhodium, nickel, or platinum.

2. Method (M1) according to claim 1, wherein
X is Cl.

3. Method (M1) according to claim 1 or 2, wherein
R1 is selected from the group consisting of C₁₋₅ alkyl, C₃₋₆ cycloalkyl, C(O)-O-C₁₋₄ alkyl, CH=CH₂, benzyl and phenyl;
said C₁₋₅ alkyl of R1 is unsubstituted or substituted with 1, 2, 3, 4 or 5 identical of different substituents selected from the group consisting of halogen, O-C(O)-CH₃, O-C₁₋₅ alkyl, S-C₁₋₅ alkyl, S(O)-C₁₋₅ alkyl, S(O₂)-C₁₋₅ alkyl, O-C₁₋₄ alkylen-O-C₁₋₄ alkyl, C₃₋₆ cycloalkyl and 1,2,4-triazolyl;
said benzyl and said phenyl of R1 are independently from each other unsubstituted or substituted with 1, 2, 3, 4 or 5 identical of different substituents selected from the group consisting of halogen, C₁₋₂ alkoxy, NO₂ and CN.

4. Method (M1) according to one or more of claims 1 to 3, wherein
Y is selected from the group consisting of C₁₋₆ alkoxy, NHR4 and N(R4)R5;
R4 and R5 are identical or different and independently from each other C₁₋₆ alkyl, or represent together a 1,4-butylene or a 1,5-pentylene chain.

5. Method (M1) according to one or more of claims 1 to 4, wherein
compound (HyS) is selected from the group consisting of hydrogen, cyclohexene, formic acid, salts of formic acid, and mixtures thereof.

6. Method (M1) according to one or more of claims 1 to 5, wherein
salts of formic acid salts are selected from the group consisting of
[N(R10)(R11)(R12)(R13)]⁺[HCOO]⁻, hydrazinium formate, sodium formate and potassium formate;
R10, R11, R12 and R13 are identical or different and independently from each other selected from the group consisting of H, C₁₋₆ alkyl, and C₂₋₆ alkylene-OH;
more preferably, salts of formic acid salts are selected from the group consisting of ammonium formate, hydrazinium formate, sodium formate, potassium formate, triethylammonium formate and tributylammonium formate.

7. Method (M1) according to one or more of claims 1 to 6, wherein
catalyst (CatS1) is selected from the group consisting of palladium powder, palladium on C, palladium on Al₂O₃, ruthenium on C, ruthenium on Al₂O₃, rhodium on C, rhodium on Al₂O₃, Raney nickel, platinum powder, platinum on carbon, platinum on Al₂O₃, PtO₂, and mixtures thereof.

8. Method (M1) according to one or more of claims 1 to 7, wherein
reaction (ReacS1) is done in the presence of a base (BasS1); base (BasS1) is selected from the group consisting of N(R20)(R21)(R22), unsubstituted pyridine, pyridine substituted with 1, 2 and 3 identical or different substituents independently from each other selected from the group consisting of methyl and ethyl, salts of [N(R30)(R31)(R32)(R33)]⁺ with C₂₋₆ carboxylic acids, salts of Na and K with carbonate and bicarbonate, hydroxides of Na, K and Ca, and mixtures thereof;
R20, R21 and R22 are identical or different and independently from each other selected from the group consisting of H, C₁₋₆ alkyl and C₂₋₆ alkylene-OH;
R30, R31, R32 and R33 are identical or different and independently from each other H or C₁₋₆ alkyl.

9. Method (M1) according to one or more of claims 1 to 8, wherein
method (M1) comprises furthermore a step (S2), step (S2) is done before step (S1), in step (S2) compound of formula (II) is prepared;
step (StepS2) comprises a reaction (ReacS2);
reaction (ReacS2) is a reaction of a compound of formula (III) with a compound (HalS2);
compound of formula (III) is selected from the group consisting of compound of formula (IIIa), compound of formula (IIIb) and mixtures thereof; 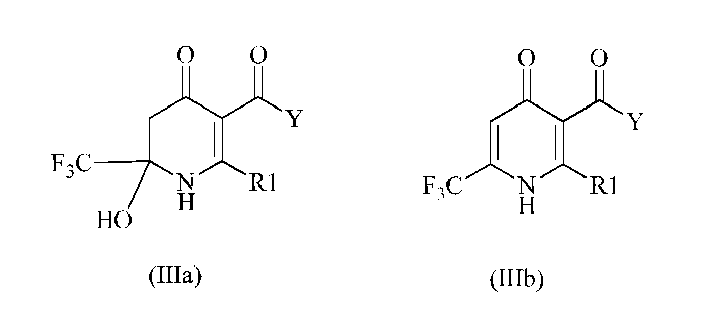 with R1 and Y as defined in claim 1;
compound (HalS2) is selected from the group consisting of POCl₃, PCl₃, PCl₅, SOCl₂, COCl₂, diphosgene, triphosgene, (COCl)₂, 2,4,6-trichloro-1,3,5-triazine, POBr₃, PBr₃, PBr₅, Ph₃PBr₂, Br₂ with P(OPh)₃, P₄O₁₀ together with NaBr, N-bromosuccinimide together with PPh₃, and mixtures thereof.

10. Method (M1) according to claim 9, wherein
reaction (ReacS2) is done in the presence of a base (BasS2); base (BasS2) is selected from the group consisting of N,N-di-C₁₋₄ alkyl aniline, N(R40)(R41)(R42),
N-C₁₋₄ alkyl morpholine, unsubstituted pyridine, pyridine substituted with 1, 2 and 3 identical or different substituents independently from each other selected from the group consisting of methyl and ethyl, and mixtures thereof;
R40, R41 and R42 are identical or different and independently from each other C₁₋₆ alkyl.

11. Method (M1) according to claim 9 or 10, wherein
method (M1) comprises furthermore a step (S3), step (S3) is done before step (S2), in step (S3) compound of formula (III) is prepared;
step (StepS3) comprises a reaction (ReacS3);
reaction (ReacS3) is a reaction of a compound of formula (IV) with a compound (AMS); compound of formula (IV) is selected from the group consisting of compound of formula (IVa), compound of formula (IVb), compound of formula (IVc), a hydrate of compound of formula (IVa), of compound of formula (IVb) and of compound of formula (IVc), and mixtures thereof; 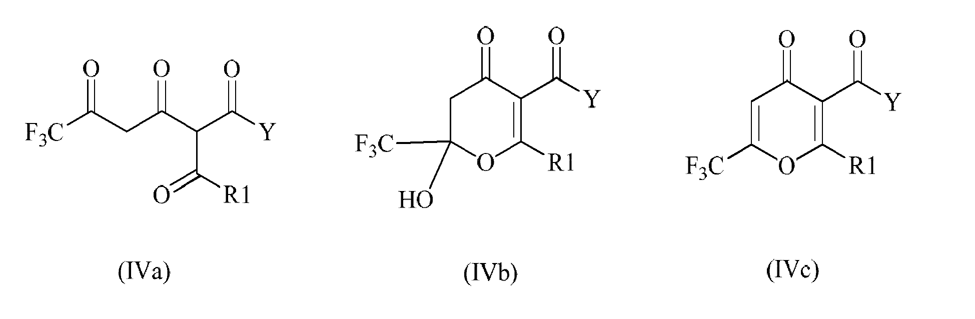 with R1 and Y as defined in claim 1;
compound (AMS) is selected from the group consisting of NH₃, sulfamic acid, urea, formamide, NH₂-C(O)-C₁₋₄ alkyl, ammonium carbonate, ammonium bicarbonate, ammonium salts of C₁₋₆ carboxylic acids, ammonium chloride, ammonium bromide, and mixtures thereof.

12. Method (M1) according to claim 11, wherein
compound (AMS) is selected from the group consisting of NH₃, formamide, ammonium carbonate, ammonium bicarbonate, ammonium formate, ammonium acetate, ammonium chloride and mixtures thereof.

13. Method (M1) according to claim 11 or 12, wherein
method (M1) comprises furthermore a step (S4), step (S4) is done before step (S3), in step (S4) compound of formula (IV) is prepared;
step (StepS4) comprises a reaction (ReacS4);
reaction (ReacS4) is a reaction of a compound of formula (V) with a compound of formula (VI) facilitated by the use of a compound (SalS4); 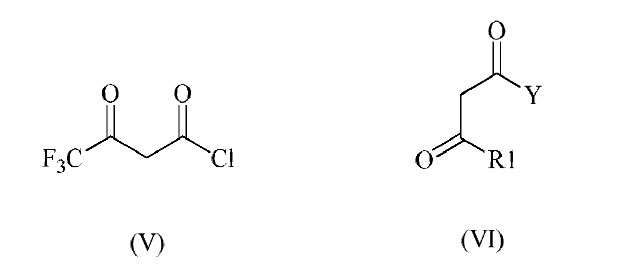 with R1 and Y as defined in claim 1;
compound (SalS4) is selected from the group consisting of NaH, Na(O(C₁₋₆ alkyl)), CaH₂, CaCl₂, MgCl₂, MgBr₂, MgI₂, Mg(O(C₁₋₆ alkyl))2, TiCl₄, TiCl₂(O(C₁₋₆ alkyl))2, TiCl₃(O(C₁₋₆ alkyl)), ZnCl₂, ZnBr₂, ZnI₂, SnCl₄, SnBr₄, AlCl₃, AlBr₃, Al(O(C₁₋₆ alkyl))3, and mixture thereof.

14. Method (M1) according to claim 13, wherein
compound (SalS4) is selected from the group consisting of Na(O(C₁₋₆ alkyl)), CaH₂, CaCl₂, MgCl₂, MgBr₂, Mg(O(C₁₋₆ alkyl))₂, TiCl₄, TiCl₂(O(C₁₋₆ alkyl))2, TiCl₃(O(C₁₋₆ alkyl)), ZnCl₂, Aids, AlBr₃, Al(O(C₁₋₆ alkyl))₃, and mixture thereof.

15. Method (M1) according to claim 13 or 14, wherein
reaction (ReacS4) is facilitated by the use of a base (BasS4); base (BasS4) is selected from the group consisting of N(R50)(R51)(R52), unsubstituted pyridine, pyridine substituted with 1, 2 and 3 identical or different substituents independently from each other selected from the group consisting of methyl and ethyl, N-C₁₋₄ alkyl morpholine, and mixtures thereof;
R50, R51 and R52 are identical or different and independently from each other C₁₋₆ alkyl.

16. Method (M2) for preparation of compound of formula (II);
the method (M2) comprises the step (S2),
wherein compound of formula (II) is defined as in claim 1 and step (S2) defined as in claim 9.

17. Method (M2) according to claim 16; wherein
method (M2) comprises furthermore the step (S3), step (S3) is done before step (S2), in step (S3) compound of formula (III) is prepared;
wherein compound of formula (III) is defined as in claim 9 and step (S3) is defined as in claim 11.

18. Method (M2) according to claim 17; wherein
method (M2) comprises furthermore the step (S4), step (S4) is done before step (S3), in step (S4) compound of formula (IV) is prepared;
wherein compound of formula (IV) is defined as in claim 11 and step (S4) is defined as in claim 13.

19. Method (M3) for preparation of compound of formula (III);
the method (M3) comprises the step (S3),
wherein compound of formula (III) is defined as in claim 9 and step (S3) is defined as in claim 11.

20. Method (M3) according to claim 19; wherein
method (M3) comprises furthermore the step (S4), step (S4) is done before step (S3), in step (S4) compound of formula (IV) is prepared;
wherein compound of formula (IV) is defined as in claim 11 and step (S4) is defined as in claim 13.

21. Method (M4) for preparation of compound of formula (IV);
the method (M4) comprises the step (S4),
wherein compound of formula (IV) is defined as in claim 11 and step (S4) is defined as in claim 13.

22. Compound of formula (IV), compound of formula (III) and compound of formula (II);
wherein compound of formula (IV) is defined as in claim 11, compound of formula (III) is defined as in claim 9 and compound of formula (II) is defined as in claim 1.

23. Compound of formula (IV), compound of formula (III) and compound of formula (II) according to claim 22;
wherein
compound of formula (IV) is compound of formula (IVc-1);
compound of formula (III) is compound of formula (IIIa-1) or compound of formula (IIIb-1); and
compound of formula (II) is compound of formula (II-1).

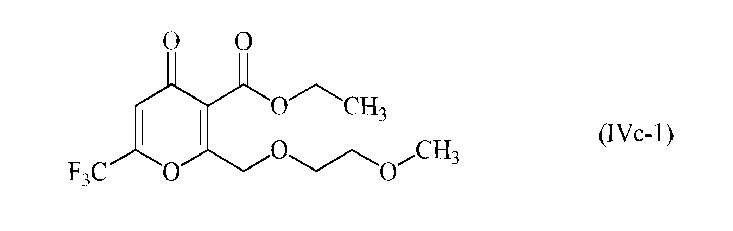

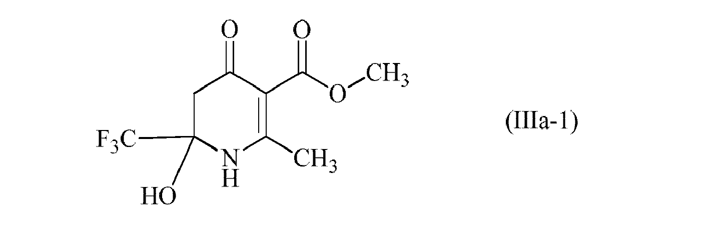

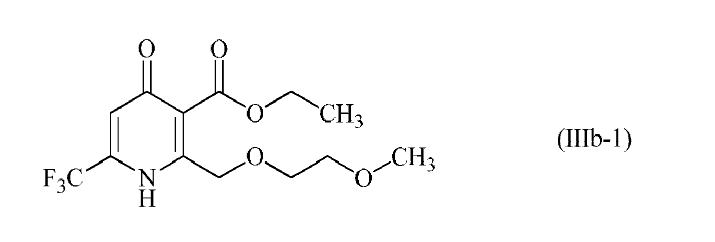

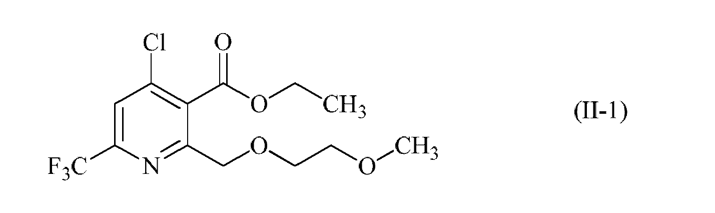

24. Use of anyone of compound of formula (IV), compound of formula (III), compound of formula (II) and compound of formula (I) for the preparation of pharmaceutical, chemical or agro-chemical products,
with compound of formula (IV) as defined in claim 11,
with compound of formula (III) as defined in claim 9, and
with compound of formula (II) and compound of formula (I) as defined in claim 1;
wherein
compound of formula (IV) has been prepared according to claim 21,
compound of formula (III) has been prepared according to claim 19,
compound of formula (II) has been prepared according to claim 16, and
compound of formula (I) has been prepared according to claim 1.
